# EUROPEAN PATENT APPLICATION

(11) **EP 1 589 032 A1**
(43) Date of publication of application: **26.10.2005**
(21) Application number: 03815554.5
(22) Date of filing: 29.01.2003
(51) Int. Cl.: C07K 14/495, C12N 15/31, C12N 15/81

(54) **GENETICALLY-MODIFIED YEASTS WHICH CAN FLOAT IN A LIQUID MEDIUM, PRODUCTION METHOD THEREOF AND USE OF SAME**

(71) Applicant: Osborne Distribuidora S.A., 28034 Madrid (ES); Universidad Pablo de Olavide, 41013 Sevilla (ES)
(72) Inventor: FIDALGO MERINO, Manuel, E-41013 Sevilla (ES); IBEAS CORCELLES, Jose Ignacio, E-41013 Sevilla (ES); RAMOS BARRALES, Ramon, E-41013 Sevilla (ES); JIMENEZ MARTINEZ, Juan, E-41013 Sevilla (ES)
(74) Representative: Carvajal y Urquijo, Isabel
(86) International application number: PCT/ES2003/000048
(87) International publication number: WO 2004/067565

(57) **Abstract**

The object of the present invention is new genetically modified yeasts with the capability to form a biofilm at the liquid/air interphase, as well as a process for obtaining fungi and yeasts with the capability to form a biofilm with those features, and the use thereof.

## Description

The object of the present invention is new genetically modified yeasts with capability to form a biofilm at the liquid/air interphase, as well as a process for obtaining fungi and yeasts with the capability to form a biofilm with those features, and the use thereof.

The biofilms formed at the liquid-air interphase by fungi or yeasts with said innate or acquired capability, and their different applications, especially indicated in the production of biofilms for elaborating biologically aged sherry wine, are also object of this patent.

It is known that certain *S. cerevisiae* strains have the capability to grow on the surface of wines, forming a biofilm known as flor in the field of producing biologically aged sherry wine.

To that regard, patent WO02/14493 discloses the capability of fungi and yeasts to form a biofilm on solid or semi-solid surfaces and its applications, and more specifically, the design of a model for studying biofilms produced by *S. cerevisiae* and for studying drugs positively and negatively affecting their growth, as well as the identification of genes necessary for forming the biofilm.

Studies carried out by *A. Bakalinsky* and *S. Zara,* of the University of Oregon and the University of Sassari, respectively, were focused on the role of the HSP12 gene in the formation and maintenance of biofilms produced by certain *S. cerevisiae* strains, as well as the possible connection of the altered metabolism of lipids and the formation of said biofilm (*"HSP12 is essential for biofilm formation by Sardinian wine strain of S. cerevisiae")*.

Researchers *S. Kuchin, VK Vyas,* and *M. Carlson,* of the University of Columbia, have recently published an article which details the role of Snf1 protein kinase of *S. cerevisiae* in the regulation of transcription of the FLO11 gene *(Snf1 protein kinase and the repressors NRg1 and NRg2 regulate FLO11, haploid invasive growth, and diploid pseudohypal differentiation").*

There are also numerous scientific works disclosing drawbacks and applications related to biofilm formation in four main aspects:
**a)** Those related to biologically aged wine. In this sense, sherry wine flor is a biofilm of yeasts of the *Saccharomyces* genus preventing its oxidation in the aging cask, and which provides it with its organoleptic features through its metabolism. Maintaining and reproducing this biofilm is many times beyond the control of the enologist, as is disclosed in scientific works:
   Ibeas J. I, Lozano I, Perdigones F, Jiménez J. (1997). Effects of ethanol and temperature on the biological aging of sherry wines. Am. J. Enol. Vitic. **48:** 71-74.
   lbeas J. I, Lozano I, Perdigones F, Jiménez J. (1997). Population dynamic of flor yeasts during the biological aging of sherry wines. Am. J. Enol. Vitic. **48:** 75-79.
   Martínez P, Codon AC, Perez L, Benitez T. (1995). Physiological and molecular characterization of flor yeasts: polymorphism of flor yeast populations. Yeast. **14:** 1399-1411.
   Martinez P, Perez L, Benitez T. (1997). Factors which affect velum formation by flor yeasts isolate from Sherry wine. System. Appl. Microbiol. **20:** 154-157.
   Martínez P, Perez L, Benitez T. (1997). Evolution of flor yeast population during the biological aging of fino Sherry wine. Am. J. Enol. Vitic. **48:** 130-138.
**b)** Filamentous yeasts and fungi are microorganisms of applied interest by themselves, as a medium for the industrial production of proteins and other compounds of applied interest, and as a microorganism performing transformations of foods and drinks by fermentation. When this industrial activity is carried out cultivating the fungi or yeasts in liquid media, it is difficult to separate the microorganism from this medium where it is cultivated, and any process facilitating this task noticeably increases the efficacy of the industrial process.
   Riesemberg D, and Guthke R. (1999). High-cell-density cultivation of microorganisms. Appl. Microbiol. Biotechnol. **51:** 422-430.
   Da Matta VM, Medronho R de A. (2000). A new method for yeast recovery in batch ethanol fermentations: filter aid filtration followed by separation of yeast from filter aid using hydrocyclones. Bioseparation **9:** 43-53.
   Domingues L, Teixeira JA, Lima N. (1999). Construction of a flocculant Saccharomyces cerevisiae fermenting lactose. Appl. Microbiol. Biotechnol. **51:** 621-6.
   Domingues L, Teixeira JA, Penttila M, Lima N. (2002). Construction of a flocculant Saccharomyces cerevisiae strain secreting high levels of Aspergillus niger beta-galactosidase. Appl. Microbiol. Biotechnol. **58:** 645-50.
**c)** Pathogenic fungi, bacteria and yeasts are particularly resistant to antibiotics due to biofilm formation in the infected mucosae and tissues.
   Singh PK, Parsek MR, Greenberg EP, Welsh MJ. (2002). A component of innate immunity prevents bacterial biofilm development. Nature **30:** 552-5.
   Prouty AM, Schwesinger WH, Gunn JS. (2002). Biofilm formation and interaction with the surfaces of gallstones by Salmonella spp. Infect. Immun. **70:** 2640-9.
   Donlan RM, Costerton JW. (2002). Biofilms: survival mechanisms of clinically relevant microorganisms. Clin. Microbiol. Rev. **15:** 167-93.
   Wilson M. (2001). Bacterial biofilms and human disease. Sci Prog **84:** 235-54. Donlan RM. (2001). Biofilm formation: a clinically relevant microbiological process. Clin. Infect. Dis. **33:** 1387-92
   Chandra J, Kuhn DM, Mukherjee PK, Hoyer LL, McCormick T, Ghannoum MA. (2001). Biofilm formation by the fungal pathogen Candida albicans: development, architecture and drug resistance. J. Bacteriol. **183:** 5385-94.
   One objective of the pharmaceutical industry is to know the proteins involved in biofilm development to produce new specific antibiotics against them and to control this resistance, as is disclosed in the following scientific review:
   Costerton et al. (1999). Bacterial biofilms: A common cause of persistent infections. Science **284:** 1318-1322.
**d)** Control of biofilms in general is a powerful working tool in water depuration systems and for the control of other aquatic environments related to the environment, as is disclosed in the following scientific review:
   Winpenny et al. (2000). Heterogeneity in biofilms. FEMS microb. rev. **24:** 661-671.

The object of the present invention consists of achieving growth of a biofilm at the liquid-air interphase using fungi or yeasts having said capability, or else fungi and yeasts which do not naturally have this property and it is conferred to them by means of the processes which are also object of this patent, and controlling the amount and quality of said biofilm in fungi and yeasts which they originally lack, or which already have this biofilm formation characteristic.

For better understanding, a description is made below with reference to the drawings, in which:
Figure 1 shows plasmid pBSFLO1-13 resulting from cloning FLO1-13 fragments into the EcoRv (KpnI-SacI) site of the pBSSK vector.
Figure 2 shows the generation of plasmid pBSFLO1-42.
Figure 3 shows the generation of plasmid pBSFLO1-1342.
Figure 4 shows the generation of plasmid pFLO1-1342-KANloxP.
Figure 5 shows the generation of plasmid pDBGal1.
Figure 6 shows the generation of plasmid pFLT.
Figure 7 shows the generation of plasmid pFLT-FLO11.
Figure 8 schematically shows the different steps followed in the production of compounds using fungi or yeasts.
Figure 9 shows the effect caused by deletion of the FLO11 gene in a strain with the capability to form a biofilm at the liquid-air interphase.

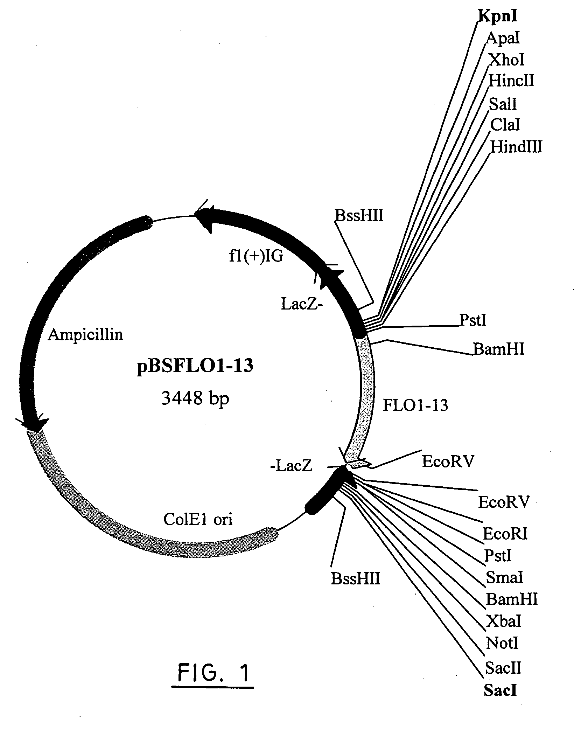

The advantages of using the system object of this patent are disclosed according to the field of application, and they are the following:
**a)** Elaboration of sherry wines: Disruption of the FLO1 gene in a strain forming a biofilm at the liquid-air interphase causes the biofilm formed by the strain to provide better isolation of the wine contained in the cask, thus preventing its oxidation, it accelerates metabolism of the wine and, therefore, the occurrence of the organoleptic components typical of sherry wine, it improves stability of this biofilm in the summer months and, in the remote case of losing this biofilm, it will allow a fast regeneration from the surviving yeasts or from inocula grown in the laboratory and introduced in the casks.
   Transformation with the plasmid containing the cloned FLO11-F gene of a yeast forming a biofilm at the liquid-air interphase (object of the patent) will allow non-biofilm-forming fungi and yeasts to acquire this capability. Combined use of the two previously mentioned processes will increase the capability to form a biofilm in the strains not having said capability, thus being able to increase variability of strains to use in the production of sherry wines, which will allow improving or varying the production process.
**b)** Separation of fungi and yeasts from the culture medium in industrial processes: The use of the plasmids object of this patent would allow obtaining strains carrying out the fermenting or metabolic process in the form of biofilm at the liquid-air interphase, so that once fermentation is carried out, the medium used by the lower portion of the fermenter is removed and new medium is pumped in, without needing to withdraw the fungus or yeast, and without affecting its growth, thus achieving a continuous high-yield culture (figure 1).
**c)** Use of *S. cerevisiae* as a model organism for studying the biofilm formation at the liquid-air interphase.
   *S. cerevisiae* is the most studied fungus from all points of view. Since biofilm formation in other fungi, and even in bacteria, shares many common elements with *S. cerevisiae,* use of the latter as a model organism can provide a great amount of information.

### DESCRIPTION OF THE INVENTION

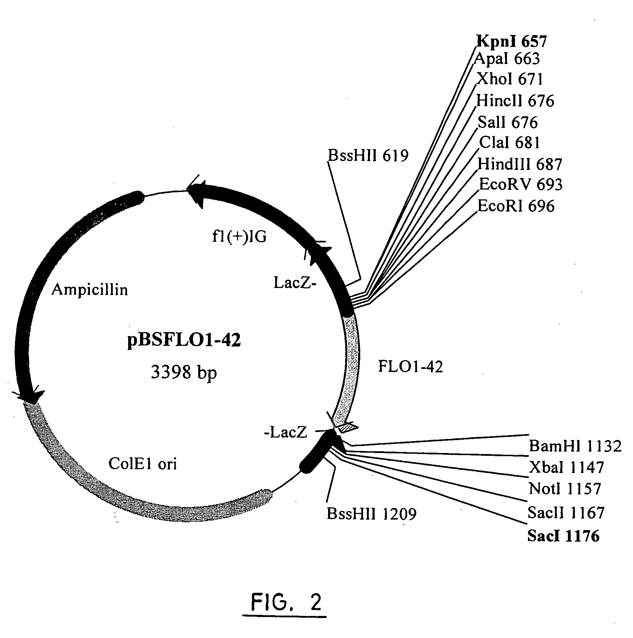

The invention is based on the authors' discovery that the capability to form a biofilm on the surface of the wine, known as flor in the field of biologically aged sherry wine production, depends on modifications in the FLO11 gene of yeasts, and that, therefore, this feature of growing and remaining on the liquid surface can be controlled with modifications in the corresponding Flo11p protein (figure 2).

The main modifications allowing the inventors to induce the formation of a biofilm at the liquid-air interphase are of three types:
1) Removal of proteins competing for the same actuations areas as Flo11p in yeast, such as the homologous Flo1p protein or the FLO1 gene encoding it.
2) Increasing the amount of Flo11p protein that the cell has, which is achieved, among other ways, by means of the expression with a very powerful promoter, or by means of deletion of the elements limiting or inhibiting expression of its own promoter.
3) Flo11p is formed by repetitions of two types of peptides: type a), represented by amino acid sequence P-V/A-P-T-P-S-S-S-T-T-E-S-S-S-A and analogous sequences, and type b) represented by sequence P-V/P-T/S/F-S-S-T/S-T-E-S-S-S/V-A/V and analogous sequences. Growth at the liquid-air interphase is also achieved by expressing variants of Flo11p protein which alternate these two basic repetition types, preventing the existence of more than two repetitions of the same type together, as well as increasing the number of repetitions of the protein.

Each one of these elements alone or combined allows a yeast to form a biofilm at the liquid-air interphase.

The experiments listed below are described as support of particular aspects of the invention, and by no means to limit the scope thereof. Said experiments were developed in the steps detailed below:

### 1) Disruption of the FLO1 gene in the MY138 strain

**1.1** To carry out the disruption of the FLO1 gene, which encodes pFLO1, a protein having a cell wall being highly homologous to FLO11, FL01-13 (-222 to 245 with regard to +1 of ORF) and FL01-42 (3135 to 3551) fragments corresponding to ends 5' and 3', respectively, of said gene were cloned into the pBSSK vector by PCR. To do this, the FL01-13 fragment is amplified by PCR with Taq using FL01-1 and FL01-3 primers, and the following program:

FLO1-1 TGGTAAACAGCTCTGCAGCAGGG

FLO1-3 TCAATCGAGATATCAGTTTGTCCTCC

### Program:

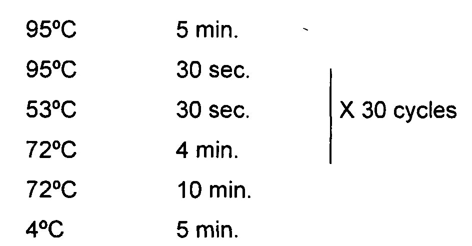

FL01-13 was cloned into the EcoRV (KpnI-SacI) site of pBSSK vector, generating plasmid pBSFL01-13 (figure 1).

1.2. The FL01-42 fragment is amplified by PCR with Taq using the FL01-2 and FL01-4 primers and the same program as in the previous case.

FLO1-4 TGAACCATGAATTCGTACTTTTACTTCG

FLO1-2 ACGGATCCAGCTGAACTCGTTTCG

It was cut with EcoRI and BamHI and cloned into pBSSK at EcoRI/BamHI sites, generating plasmid pBSFLO1-42 (figure 2).

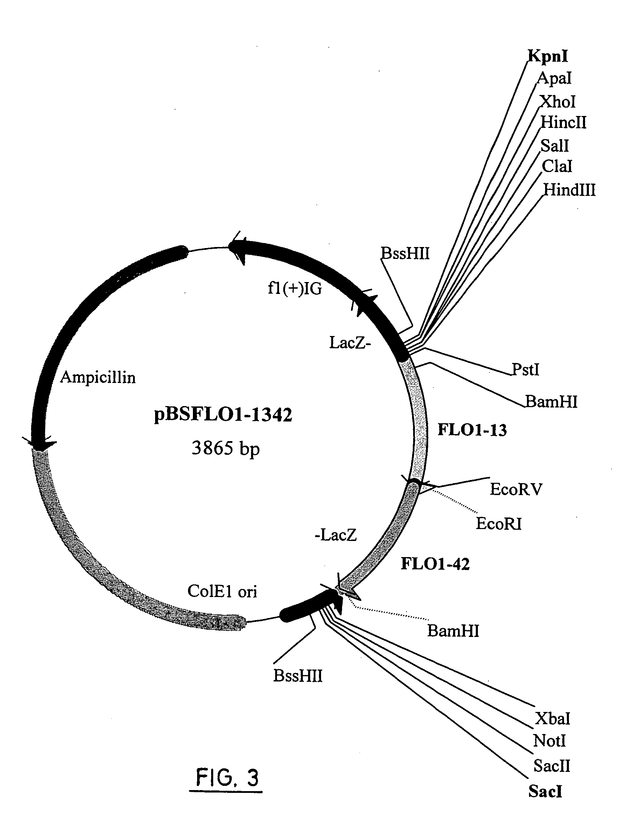

**1.3.** Then, the FLO1-42 fragment was extracted from plasmid pBSFLO1-42 with EcoRI and Xbal enzymes, and it was cloned into plasmid pBSFLO1-1342 at the EcoRI and Xbal sites, generating plasmid pBSFLO1-1342 (figure 3).

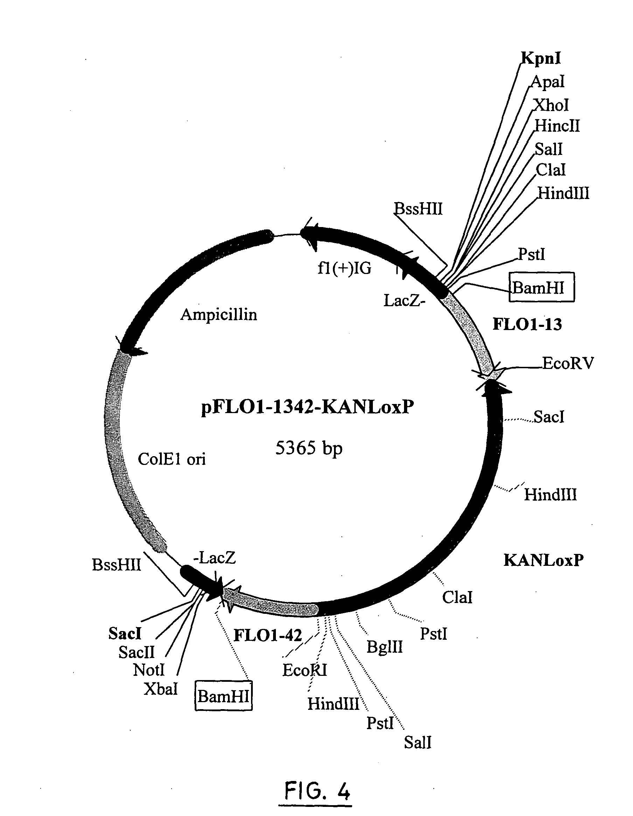

**1.4.** The fragment corresponding to the KAN gene (confers dominant geneticin resistance) flanked by the LoxP sequences originating from plasmid pUG6 *(Guldener et al 1996),* cut with EcorRV and Pvull, was cloned into plasmid pBSFLO1-1342 between the FL01-13 and FL01-42 regions, at the EcoRV site, generating the plasmid called pFLO1-1342-KANIoxP (figure 4).

*Guldener U, Heck S, Fielder T, Beinhauer U, and Hegemann JH. (1996). A new efficient gene disruption cassette for repeated use in budding yeast. Nucleic Acids Res. 24: 2519-24.*

**1.5** Digestion of the previous construct with BamHI generates a 2500-base pair fragment. This fragment was transformed into the MY138 strain by the Lithium Acetate method (Ito et al 1983), and geneticin resistant transformants were selected on rich medium plates containing 200 µg/ml of geneticin. Several selected transformants were re-checked for the geneticin resistance phenotype, and a PCR reaction was subsequently carried out using the FL01-1 and FL01-2 primers and the previously described cycles and a southern blot using the FL01-13 and FL01-42 fragments as a probe to check if the transformed fragment had been integrated and, therefore, disrupted only the FLO1 gene.

**1.6**. To remove the DNA fragments not pertaining to the yeast and to thus achieve a GRAS strain, a disruptant selected with plasmid Yep32cre-cyh *(Ito H., Fukada Y., Murata K., and Kimura A. (1983). J. Bacteriol. **153,** 163),* which carries the sequence corresponding to cre recombinase under the control of the GAL1 promoter, was transformed. Once the transformants are selected by means of growth on YNB medium plates containing 200 µg/ml of cycloheximide, these were grown in medium containing galactose as a carbon source to induce cre recombinase expression. Cycloheximide-sensitive (they have lost plasmid Yep32cre-cyh) and geneticin-sensitive (they have lost the Kan gene of the fragment used for FLO1 disruption) colonies were subsequently selected. The loss of said elements was subsequently checked by means of PCR reactions using the FL01-1 and FL01-2 primers as was done previously, and by means of southern blot using fragments corresponding to the Kan, URA3 gene and the FLO1-13 fragment as a probe. Once a strain was selected complying with all those conditions, the process was repeated to remove the second copy of the FLO1 gene which the MY138 strain has, thus obtaining a GRAS strain with double disruption of the FLO1 gene, which is called OSB101.

Application of the disruption of the FLO1 gene is the improvement of the capability to form flor of strains already having said capability.

**2) and 3) Construction of plasmid pFLT-FLO11-F conferring the capability to form a biofilm at the liquid-air interphase to those strains not having this capability by means of the increase of the protein amount and production of a variant of this FLO11-Fp protein present in strain MY138 forming the biofilm at the liquid-air interphase.**

**2.1.** For the construction of plasmid pFLT-FLO11-F, the promoter of the FL011-F gene (sequence in attached document) was amplified by PCR using the Pfu enzyme, genomic DNA extracted from the 133d strain by means of the method disclosed by Hoffman & Winston, Gene 57, 267-272 (1987), and the FLO11-P5 and FLO11-P6 primers.

FLO11-P5: ATTGAATTCCTAAATTCTTTACAGATGACATCTGG

FLO11-P6: GTAGAATTCGTCTTTGGATAGTGTGCGTATATGG

### The program used is:

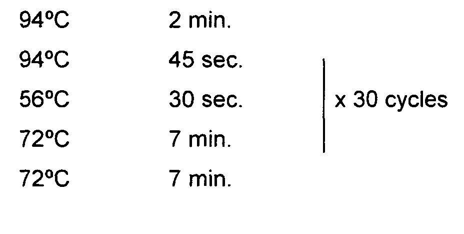

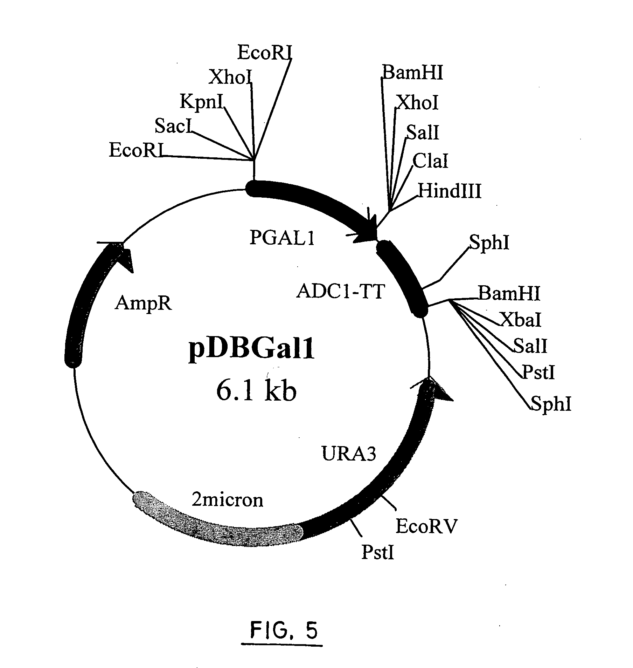

**2.2** The 3.1 Kb fragment obtained was digested with the EcoRI enzyme and was cloned into the pRS316 vector. The resulting plasmid is called pRSPFLO11. Then, the TADH terminator of plasmid pDBGal1 (figure 5) was cloned by means of HindIII-Xbal digestion and blunt-end reaction into plasmid pRSPFLO11.

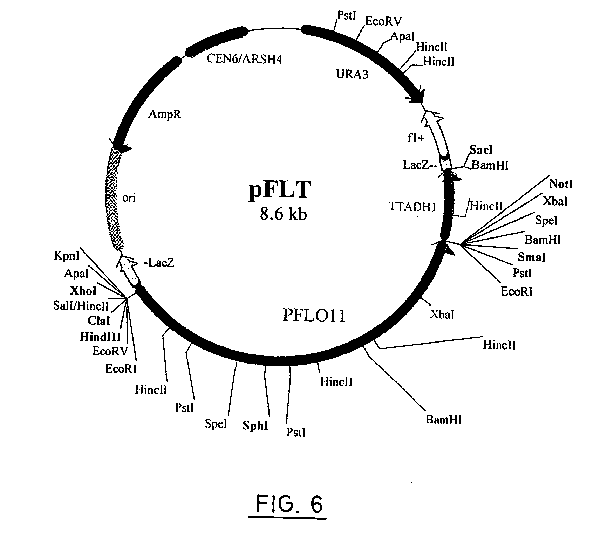

The resulting plasmid was called pFLT (figure 6).

**2.3.** The fragment corresponding to the FLO11-F gene (5 Kb) previously amplified by PCR using the Pfu enzyme, genomic DNA of the 133d strain and the FLO11-END5 and FLO11-END3 primers, was cloned into vector pBSSK+.

FLO11-END5: GCGAATTCTCAAAATCCATATACGCACACTATGC

FLO11-END3: GCTCTAGACTTATGGCAGCGAAAACAGAATGGTGG

The program used is:

| | |
|---|---|
| 94°C | 2 min. |
| 94°C | 45 sec. |
| 53°C | 30 sec. (increase 0.5°C/cycle) |
| 72°C | 9 min. |
| Number of cycles: 10 | |
| 94°C | 45 sec. |
| 58°C | 30 sec. |
| 72°C | 9 min. (increase 5 sec./cycle) |
| Number of cycles: 20 | |
| 72°C | 7 min. |

A 5 Kb amplified DNA size is obtained

The resulting plasmid is called pFLO11-C.

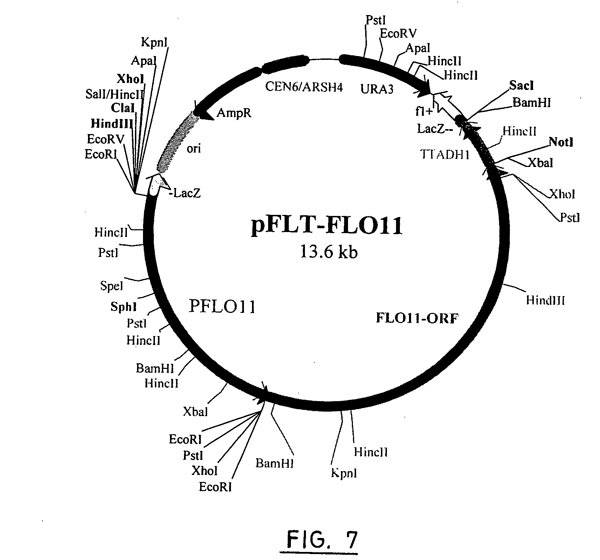

**2.4.** The FLO11 fragment was finally extracted from plasmid pFLO11-C by means of digestion with Apal (and reaction to make blunt ends) and NotI, and it was cloned into plasmid pFLT at SmaI-NotI. The resulting plasmid is called pFLT-FLO11 (figure 7).

Application of the pFLT-FLO11 construct is to confer the capability to form a biofilm at the liquid-air interphase to strains lacking said capability, and to improve this capability in those already having it.

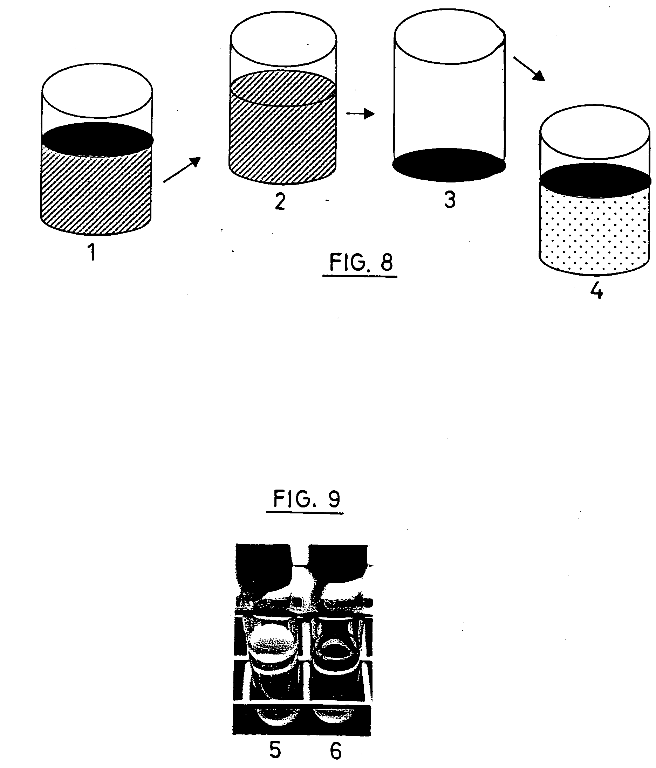

Figure 8 shows the different steps in producing compounds using fungi and yeasts, taking advantage of the capability to form a biofilm.

In said figure: 1 represents the growth of the culture, 2 the transfer of the metabolized medium or substrate, 3 the strained biofilm, and 4 the filling with new medium or substrate.

Figure 9 allows seeing the effect of the deletion of the FLO11 gene in a strain with the capability to form a biofilm at the liquid-air interphase, 5 corresponding to the 133d strain and 6 to 133dFLO11 strain.

GENE SEQUENCES USED
FLO1-1000 until the end of the open reading frame
AAAAAAAAGTGCATTTATTTAGGTAAGTCTCATTACCTAAACGCCAGTTT
GTTTCACGTAATTGGTAACGATGAGGGAACCGCAGTAGAAAAAACTTTC
ATTCACAAACGATTAAAGTGTTATGCTAGCCAGTTTCAGGCTTTTTGTTTT
ATGCAAGAGAACATTCGACTAGATGTCCAGTTAAGTGTGCGTCACTTTTC
CTACGGTGCCTCGCACATGAATGTTATCCGGCGCACGATACTTATCACCG
AAAAACCTTATTCTACGGAAAACCTTATTTACATTAAAGTTGGAAAAATT
TCCTCTTTTCCTAATAAGGTGGAGCTTTTGGCTTCCAGTATGCTTTCACG
GAATTATTTCTCATGTACATTTAGCTCCATTTCCAGTGCCTCCGATAGGGA
GGCATCATGGTACTACCGTGACGGAGAATACGTAGGCTGACTTTTTCGTC
AGTTTGTTGTCCGTTTACAAAATTGGTGAATGAATTCTAGCCTTCCTCTGC
TCATTAATTGCCCTCACAAGAATTTGGAAGTGCGTAGACAGGTAAAAGAT
TGTACTACAGAGGTATTGTGGAACCTTCTACAGTACTTCGGAATACACCT
AAAAGGTTGTTGGATGCTAAATTTAGCAAAAGTCTTTTTAGCTCACTATT
AGGCTTGTTAAAGTCTGAAATTGTTGAAAGGCACTCAAAAAGATAAATC
AACAATCAGCATTAACGGCACAGTTGAAAGAGTCACCCACTTGAAATTA
GCTCGGTTATCAAATATAATTATCTCTGGTAAAGAGCTCTGCAGCAGGGT
TAATCTATTCGCATACTTACGCTGTAGGAACATTTTATTATTAGGATCCGA
CTACTGCCTACATATTTATTCGGAAGGCATGATGTCGAAAATTTTTGAGC
TTATAAAAGGAACATATTTCACTCTTGCTCGTTGATGTAAGCTCTCTTCCG
GGTTCTTATTTTTAATTCTTGTCACCAGTAAACAGAACATCCAAAAATGA
CAATGCCTCATCGCTATATGTTTTTGGCAGTCTTTACACTTCTGGCACTAA
CTAGTGTGGCCTCAGGAGCCACAGAGGCGTGCTTACCAGCAGGCCAGAG
GAAAAGTGGGATGAATATAAATTTTTACCAGTATTCATTGAAAGATTCCT
CCACATATTCGAATGCAGCATATATGGCTTATGGATATGCCTCAAAAACC
AAACTAGGTTCTGTCGGAGGACAAACTGATATCTCGATTGATTATAATAT

TCCCTGTGTTAGTTCATCAGGCACATTTCCTTGTCCTCAAGAAGATTCCTA
TGGAAACTGGGGATGCAAAGGAATGGGTGCTTGTTCTAATAGTCAAGGA
ATTGCATACTGGAGTACTGATTTATTTGGTTTCTATACTACCCCAACAAAC
GTAACCCTAGAAATGACAGGTTATTTTTACCACCACAGACGGGTTCTTA
CACATTCAAGTTTGCTACAGTTGACGACTCTGCAATTCTATCAGTAGGTG
GTGCAACCGCGTTCAACTGTTGTGCTCAACAGCAACCGCCGATCACATCA
ACGAACTTTACCATTGACGGTATCAAGCCATGGGTGGAAGTTTGCCACC
TAATATCGAAGGAACCGTCTATATGTACGCTGGCTACTATTATCCAATGA
AGGTTGTTTACTCGAACGCTGTTTCTTGGGGTACACTTCCAATTAGTGTGA
CACTTCCAGATGGTACCACTGTAAGTGATGACTTCGAAGGGTACGTCTAT
TCCTTTGACGATGACCTAAGTCAATCTAACTGTACTGTCCCTGACCCTTCA
AATTATGCTGTCAGTACCACTACAACTACAACGGAACCATGGACCGGTAC
TTTCACTTCTACATCTACTGAAATGACCACCGTCACCGGTACCAACGGCG
TTCCAACTGACGAAACCGTCATTGTCATCAGAACTCCAACAACTGCTAGC
ACCATCATAACTACAACTGAGCCATGGAACAGCACTTTTACCTCTACTTC
TACCGAATTGACCACAGTCACTGGCACCAATGGTGTACGAACTGACGAA
ACCATCATTGTAATCAGAACACCAACAACAGCCACTACTGCCATAACTAC
AACTGAGCCATGGAACAGCACTTTTACCTCTACTTCTACCGAATTGACCA
CAGTCACCGGTACCAATGGTTTGCCAACTGATGAGACCATCATTGTCATC
AGAACACCAACAACAGCCACTACTGCCATGACTACAACTCAGCCATGGA
ACGACACTTTTACCTCTACTTCTACCGAATTGACCACAGTCACCGGTACC
AATGGTTTGCCAACTGATGAGACCATCATTGTCATCAGAACACCAACAAC
AGCCACTACTGCCATGACTACAACTCAGCCATGGAACGACACTTTTACCT
CTACTTCTACCGAATTGACCACAGTCACCGGTACCAATGGTTTGCCAACT
GATGAGACCATCATTGTCATCAGAACACCAACAACAGCCACTACTGCCAT
GACTACAACTCAGCCATGGAACGACACTTTTACCTCTACATCCACTGAAA
TCACCACCGTCACCGGTACCAATGGTTTGCCAACTGATGAGACCATCATT
GTCATCAGAACACCAACAACAGCCACTACTGCCATGACTACACCTCAGCC
ATGAACGACACTTTTACCTCTACATCCACTGAAATGACCACCGTCACCGG
TACCAACGGTTTGCCAACTGATGAAACCATCATTGTCATCAGAACACCAA

CAACAGCCACTACTGCCATAACTACAACTGAGCCATGGAACAGCACTTTT
ACCTCTACATCCACTGAAATGACCACCGTCACCGGTACCAACGGTTTGCC
AACTGATGAAACCATCATTGTCATCAGAACACCAACAACAGCCACTACTG
CCATAACTACAACTCAGCCATGGAACGACACTTTTACCTCTACATCCACT
GAAATGACCACCGTCACCGGTACCAACGGTTTGCCAACTGATGAAACCAT
CATTGTCATCAGAACACCAACAACAGCCACTACTGCCATGACTACAACTC
AGCCATGGAACGACACTTTTACCTCTACATCCACTGAAATCACCACCGTC
ACCGGTACCACCGGTTTGCCAACTGATGAGACCATCATTGTCATCAGAAC
ACCAACAACAGCCACTACTGCCATGACTACAACTCAGCCATGGAACGAC
ACTTTTACCTCTACATCCACTGAAATGACCACCGTCACCGGTACCAACGG
CGTTCCAACTGACGAAACCGTCATTGTCATCAGAACTCCAACTAGTGAAG
GTCTAATCAGCACCACCACTGAACCATGGACTGGTACTTTCACCTCTACA
TCCACTGAGATGACCACCGTCACCGGTACTAACGGTCAACCAACTGACGA
AACCGTGATTGTTATCAGAACTCCAACCAGTGAAGGTTTGGTTACAACCA
CCACTGAACCATGGACTGGTACTTTTACTTCTACATCTACTGAAATGACC
ACCATTACTGGAACCAACGGCGTTCCAACTGACGAAACCGTCATTGTCAT
CAGAACTCCAACCAGTGAAGGTCTAATCAGCACCACCACTGAACCATGG
ACTGGTACTTTTACTTCTACATCTACTGAAATGACCACCATTACTGGAACC
AATGGTCAACCAACTGACGAAACCGTTATTGTTATCAGAACTCCAACTAG
TGAAGGTCTAATCAGCACTACAACGGAACCATGGACCGGTACTTTCACTT
CTACATCTACTGAAATGACGCACGTCACCGGTACCAACGGCGTTCCAACT
GACGAAACCGTCATTGTCATCAGAACTCCAACCAGTGAAGGTCTAATCAG
CACCACCACTGAACCATGGACTGGCACTTTCACTTCGACTTCCACTGAGG
TTACCACCATCACTGGAACCAACGGTCAACCAACTGACGAAACTGTGATT
GTTATCAGAACTCCAACCAGTGAAGGTCTAATCAGCACCACCACTGAACC
ATGGACTGGTACTTTCACTTCTACATCTACTGAAATGACCACCGTCACCG
GTACTAACGGTCAACCAACTGACGAAACCGTGATTGTTATCAGAACTCCA
ACCAGTGAAGGTTTGGTTACAACCACCACTGAACCATGGACTGGTACTTT
TACTTCGACTTCCACTGAAATGTCTACTGTCACTGGAACCAATGGCTTGC
CAACTGATGAAACTGTCATTGTTGTCAAAACTCCAACTACTGCCATCTCA

TCCAGTTTGTCATCATCATCTTCAGGACAAATCACCAGCTCTATCACGTCT
TCGCGTCCAATTATTACCCCATTCTATCCTAGCAATGGAACTTCTGTGATT
TCTTCCTCAGTAATTTCTTCCTCAGTCACTTCTTCTCTATTCACTTCTTCTC
CAGTCATTTCTTCCTCAGTCATTTCTTCTTCTACAACAACCTCCACTTCTAT
ATTTTCTGAATCATCTAAATCATCCGTCATTCCAACCAGTAGTTCCACCTC
TGGTTCTTCTGAGAGCGAAACGAGTTCAGCTGGTTCTGTCTCTTCTTCCTC
TTTTATCTCTTCTGAATCATCAAAATCTCCTACATATTCTTCTTCATCATTA
CCACTTGTTACCAGTGCGACAACAAGCCAGGAAACTGCTTCTTCATTACC
ACCTGCTACCACTACAAAAACGAGCGAACAAACCACTTTGGTTACCGTGA
CATCCTGCGAGTCTCATGTGTGCACTGAATCCATCTCCCCTGCGATTGTTT
CCACAGCTACTGTTACTGTTAGCGGCGTCACAACAGAGTATACCACATGG
TGCCCTATTTCTACTACAGAGACAACAAAGCAAACCAAAGGACAACAG
AGCAAACCACAGAAACAACAAAACAAACCACGGTAGTTACAATTTCTTC
TTGTGAATCTGACGTATGCTCTAAGACTGCTTCTCCAGCCATTGTATCTAC
AAGCACTGCTACTATTAACGGCGTTACTACAGAATACACAACATGGTGTC
CTATTTCCACCACAGAATCGAGGCAACAAACAACGCTAGTTACTGTTACT
TCCTGCGAATCTGGTGTGTGTTCCGAAACTGCTTCACCTGCCATTGTTTCG
ACGGCCACGGCTACTGTGAATGATGTTGTTACGGTCTATCCTACATGGAG
GCCACAGACTGCGAATGAAGAGTCTGTCAGCTCTAAAATGAACAGTGCT
ACCGGTGAGACAACAACCAATACTTTAGCTGCTGAAACGACTACCAATA
CTGTAGCTGCTGAGACGATTACCAATACTGGAGCTGCTGAGACGAAAAC
AGTAGTCACCTCTTCGCTTTCAAGATCTAATCACGCTGAAACACAGACGG
CTTCCGCGACCGATGTGATTGGTCACAGCAGTAGTGTTGTTTCTGTATCCG
AAACTGGCAACACCAAGAGTCTAACAAGTTCCGGGTTGAGTACTATGTCG
CAACAGCCTCGTAGCACACCAGCAAGCAGCATGGTAGGATATAGTACAG
CTTCTTTAGAAATTTCAACGTATGCTGGCAGTGCCAACAGCTTACTGGCC
GGTAGTGGTTTAAGTGTCTTCATTGCGTCCTTATTGCTGGCAATTATTTAA

FLO11 promoter of flor yeast

GAATTCCTAAATTCTTTACAGATGACATCTGGGTCTAGGGAGATCATGCA
AAAAAATAAGACATCATTTATAACTTTCAACCTTCCGCTCACAGGACTAA
TTCTCGAAGACGCTACGAAAAGATGCTGAGCTTGATCATAATTCTTCAGG
CTCCCAATTCAAGAATACAATTACTTAGTGTGGGTGCTTCTGCTCAGTCTT
CGTTTCCTATCTCCACATACGAATCACTCGCTTGTTACTAATTCGAATAAT
TTACTGCTGTAAGGAGTCGTACCGCCAACTAAATCTGAATAACAATTTGG
CTGCTAGAAGTCAAAAAGTAGGCGCTGCAATGATTATGTGGTATGATCAG
ATTGTGTCGCAACATTCAGCGGGGTTTTGATTCAATGGGACCGCTGCTAA
TAAAGTGTTTAACTAGCGAATTTTGGAACACACCCGTTGACAAAAGTATA
AGGAGTCTCTTTCCGTGAGTTCCCCTCTTCCTCTCACTGCACTTCAACTAT
GCCTTATAGCAACCAAGAAGCTAGAAAATGCCAACTATTAAAAAGATAA
CCTCTTTGGAATTAAATAAAACGGAGATTATCTTGGGATCTATTTCACAA
ATTTACGGCTAATTGGAATGAACAGCGCCAAGTAGCACTGGACAAACTG
CAGAGAGTATTAGAACTTGCTTTATTCCGTATATCGTACTTTAGGTCGTCA
GTTTTACCACTTCGGTACGCAAATGAAAATAAAAAGCTCATTCACAACTT
AGACACAATTTCGCCGTTTACAAGTAGCTGAAAAGTCCATCTATCGAATT
ATGAATGATACTATTTTAAATATCGTCGTTACTGCACTCGTTTTCCACGTT
CTTTTTATCCACACATCCGATGTTTTATTTACAGTTGAGCATCTCAGGGCC
AGCGACAAATATAAACATATTATACTCAAAATGAAGCTCGTTGAAAAGA
GAATATTTCTTAACCTCTTTTACAGTGCTTTCAACACCTTTTCACCTGTGT
GCCATGTCAGATTAAGGATGCTCAACTTTTCGAATTATTATTACAAATTTC
AAAGAAATTACTAGTCCTTTTTGTCCCCTAATGTATCCCTCATTTCATACC
GTCGTTTTGGGGTTCCAACTACGTATAGCTTTCAAGTGTGGTTTTACGGTA
AGAACAACCCCCCATTGGTTGGTGCGGAATACTTTCCTATTCTCATCGAG
AGCCGAGCCATACACCTAAGGTGGACAGAAAGCTAAAATTCCAAAACAG
AAAAATCTAACATCTTTGCTCCCTTACAAGTAGGAACGCTGGCGTATCAA
CCCGTCTCCAGATTTGCCCAGCATTTCTGTAGAACACCCAATGCGAATGA

AGACTAATGAATATTGTAAAGACAAAAAATAGGAAAAGTTTAATTAGTG
ATGGTTCTCACCACCCCAAAACGGTCACTTGCATGCATTGAGGACATGCA
CCTGCAGTACTTGTTTTCTCGAATGTTTTACCATCTCTTTTTCCTTATC
TGAGCTACTGACGCAGACGGACATTGTTCATATTCGAAAGCTGTGCGG
GAAAACGGACCCTAACACGAATGTGAATGCGCTAATCAGTATTTCCACCA
CATGAAACCTGCTACTTTTTCAGTCCTCTTCGTATGCATTTCCCAAAATTC
ATTCGTAGCCTTGTCAACTTAGACTCAGTTCCACGGCGTGCAGGACGGGG
TATTATGAATAAAAGGATCCACGGGTGAGATTTGTTCTGTGTTTTAGGTA
TGGAGTTTTGTACCACAAAACTTTAGGAATACCGGATTTTGTGCCTACGC
CAGCCCCAGAGTATGTTCTCACAGCTGTAATTCCTAGTGATCTTTTCCTGG
CTCCAATAGGAACGCCGGTAGGCAAATTAAGGTTTTTTCTTCTGTTTTCT
TGACAAGAAAATGTCGCCCAAAGAGTTTCGGCCGTTATTTTCCTATCGAA
GTGGGTCCTTTTTGTCTTTAGTCCTTCTCTGGGGCTAGCGATCACTGCAAA
ATTAGGCTTCACTGGTACGAGTTAACTTTTTTCTTTTTTTTTTTGTCATC
CTTTTCTTTGGGGCTAAGAATGGACTTCCCTTTTCTTTTTCTTTGTTGCAGC
AGTGGCTTCAAAGAACTGCTGATTGCTCAAGGCAATCAGTCCGAGCGTTT
AGAAGGTGATTGTAGGCAGAAATTAACTTTGCGGTAAAAGAATGACATT
CTTTCTTAAAAGAAAAATTAGCTTTTTTTTGTCAGGCATTGCACAAACTT
TTTTATTTCTGCCTATACTCTTAAACAGATCAGTCATTCATGTTGTCTTTTT
AACGGTCGTACTGGGACATCACATGCCTTGGGATTCCGTAATTAGGTGCA
ACAATACGGGCACAACTCATTCTGCGCTATCTTCACGGACAGAACTTCTA
TTGCCTATCGGTGGTGTGATTAACAATTGGAGGCGCAGAGCTTGGAATGG
ATTTCCAATTCAATGGATTTGGAGGTATTCGTTTGTTTACTAATATTTACT
TTGAGGACATTGCCCAACCCTAAAAGTGCCTGTTCCAGAATAGAATAACA
TTATGATACGTTTTCTTGACCGCTGAGCAATTTAAAGCGATTATTAGGGT
ACGATTGTTTCTAGAGAAATGTGGGTCATCTTTTTAGGTCCGTTCTCTTCT
GATGAGGTAACCTTTACAAAAATGTCATAGAGTTACCAATTGGGATTCAA
GGCATCATCGCAATATACTTCGTTCTTTTACGGAGAAATTAAGCTCTTTCT
ACTTTGAATTAACTGTTAGACTTGTCTTATCTGAGGAATGTCCGTGTTCAA
ATTAAATAAAAATTTAGGGCAGTTTATTTACCTTAACAAATATGTTCAA

GCATGTACGTTACTGCGCTCTCTTCTAGTTCAAGAACGGATAACTCATAG
ACTTACCAGTACAAGTTGTTGAAGGGTTCCCAATTGATAAAAAGGATCT
TTTGCTTCCTGAAATAAACGTATAAAAAGCACCCTATTCATCAGTTATAC
TCCCTCATCATGTTGTGGTTCTAATTAAGAATATACTTTTGTAGGCCTCAA
AATCCATATACGCACACTATCCAAAGACGAATTC

FLO11 Open reading frame of flor yeast

ATGCAAAGACCATTTCTACTCGCTTATTTGGTCCTTTCGCTTCTATTTAAC
TCAGCTTTGGGTTTTCCAACTGCACTAGTTCCTAGAGGATCCTCCGAAGG
AACTAGCTGTAATTCTATCGTTAATGGCTGTCCCAACTTAGACTTCAATTG
GCACATGAACCAACAAACTATCATGCAGTATACTTTGGATGTGACTTCCG
TTTCTTGGGTTCAAGACAACACATACCAAATCACTATTCATGTCAAAGGT
AAAGAAAACATTGACCTAAAATATCTACGGTCTTTGAAAATCATTGGTGT
CACTGGTCCAAAAGGTACCGTCCAACTATACGGTTACAACGAAAATACCT
ATTTGATTGACAACCCAACTGATTTCACAGCCACTTTTGAAGTCTATGCC
ACACAAGATGTCAACAGCTGTCAGGTGTGGATGCCTAACTTCCAAATTCA
ATTCGAGTATTTGCAAGGTAGTGCCGCTCAATATGCAAGCTCTTGGAAAT
GGGGAACTACATCTTTTTATTTGTCTACTGGTTGTAACAACTATGACAATC
AAGGCCACTCTCAAACAGATTTCCCAGGCTTCTATTGGAACATAGATTGG
GACAACAATTGTGGCGGTACGAAGTCATCTACCACTACATCAACTAGTAC
TTCCGAGTCATCTACCACTACATCTAGCACTTCCGAGTCATCTACCACTAC
ATCAACTACCACTTCAGAATCATCTACATCATCATCAACCACCGCTCCTG
CTACACCAACCACTACCTCATGCACTAAGAAGAAAACCACCAGATCTAA
GACATGCACTAAGAAGACTACTACTCCAGTACCAACCCCATCAAGCTCTA
CTACTGAAAGTTCTTCTACTCCAGTAACCAGCTCCACCACTGAAAGCTCT
TCTGCTCCAGTACCAACTCCATCCAGCTCCACCACTGAAAGCTCTTCTGCT
CCAGCTCCAACCCCATCAAGCTCTACTACTGAAAGCTCTTCTGCTCCAGT
ATCCAGCTCTACTACTGAAAGCTCTTCTGCTCCAGTACCAACTCCATCCA
GCTCTACTACCGAAAGCTCTTCTACTCCAGTAACCAGCTCCACCACTGAA
AGCTCTTCTGCTCCAGCCCCAACCCCATCAAGCTCTACTACTGAAAGCTC
CTCTGCTCCAGTAACCAGCTCTACTACTGAAAGCTCTTCTGCTCCAGCTCC
AACCCCATCAAGCTCTACTACTGAAAGCTCCTCTGCTCCAGTATCCAGCT
CTACTACTGAAAGCTCTTCTGCTCCAGTACCAACTCCATCCAGCTCTACTA
CCGAAAGCTCTTCTACTCCAGTAACCAGCTCCACCACTGAAAGCTCTTCT

GCTCCAGCTCCAACCCCATCAAGCTCTACTACTGAAAGCTCCTCTGCTCC
AGTAACCAGCTCTACTACTGAAAGCTCTTCTGCTCCAGTATCCAGCTCCA
CCACTGAAAGCTCTGTAGCACCAGTACCAACCCCATCAAGCTCTACTACT
GAAAGCTCTTCTGCTCCAGTACCAACTCCATCCAGCTCTACTACCGAAAG
CTCTTCTACTCCAGTAACCAGCTCCACCACTGAAAGCTCTTCTGCTCCAGC
TCCAACCCCATCAAGCTCTACTACTGAAAGCTCCTCTGCTCCAGTAACCA
GCTCTACTACTGAAAGCTCTTCTGCTCCAGTACCAACTCCATCCAGCTCTA
CTACTGAAAGCTCCTCTGCTCCAGTATCCAGCTCTACTACTGAAAGCTCTT
CTGCTCCAGTACCAACTCCATCCAGCTCTACTACCGAAAGCTCTTCTACTC
CAGTAACCAGCTCCACCACTGAAAGCTCTTCTGCTCCAGCTCCAACCCCA
TCAAGCTCTACTACTGAAAGCTCCTCTGCTCCAGTAACCAGCTCTACTACT
GAAAGCTCTTCTGCTCCAGTATCCAGCTCCACCACTGAAAGCTCTGTAGC
ACCAGTACCAACCCCATCAAGCTCTACTACTGAAAGCTCTTCTGCTCCAG
TACCAACTCCATCCAGCTCTACTACCGAAAGCTCTTCTACTCCAGTAACC
AGCTCCACCACTGAAAGCTCTTCTGCTCCAGCTCCAACCCCATCAAGCTC
TACTACTGAAAGCTCCTCTGCTCCAGTAACCAGCTCTACTACTGAAAGCT
CTTCTGCTCCAGTACCAACTCCATCCAGCTCTACTACTGAAAGCTCTTCTA
CTCCAGTAACCAGCTCCACCACTGAAAGCTCTTCTGCTCCAGCTCCAACC
CCATCAAGCTCTACTACTGAAAGCTCCTCTGCTCCAGTAACCAGCTCTAC
TACTGAAAGCTCTTCTGCTCCAGTACCAACTCCATCCAGCTCTACTACTGA
AAGCTCTTCTACTCCAGTAACCAGCTCCACCACTGAAAGCTCTTCTGCTCC
AGCTCCAACCCCATCAAGCTCTACTACTGAAAGCTCCTCTGCTCCAGTAA
CCAGCTCTACTACTGAAAGCTCTTCTGCTCCAGCTCCAACCCCATCAAGC
TCTACTACTGAAAGCTCCTCTGCTCCAGTAACCAGCTCTACTACTGAAAG
CTCTTCTGCTCCAGTACCAACTCCATCCAGCTCTACTACTGAAAGCTCTTC
TGCTCCAGTATCCAGCTCCACCACTGAAAGCTCTGTAGCACCAGTACCAA
CCCCATCAAGCTCTACTACTGAAAGCTCTTCTGCTCCAGTACCAACTCCAT
CCAGCTCTACTACCGAAAGCTCTTCTACTCCAGTAACCAGCTCCACCACT
GAAAGCTCTTCTGCTCCAGCTCCAACCCCATCAAGCTCTACTACTGAAAG
CTCCTCTGCTCCAGTAACCAGCTCTACTACTGAAAGCTCTTCTGCTCAAGT

ACCAACTCCATCCAGCTCTACTACTGAAAGCTCTTCTGCTCCAGTATCCA
GCTCCACCACTGAAAGCTCTGTAGCACCAGTACCAACCCCATCAAGCTCT
ACTACTGAAAGCTCTTCTGCTCCAGTACCAACTCCATCCAGCTCTACTACC
GAAAGCTCTTCTACTCCAGTAACCAGCTCCACCACTGAAAGCTCCTCTGC
TCCAGCTCCAACTCCATCCAGCTCTACTACTGAAAGCTCTTCTGCTCCAGT
ATCCAGCTCCACCACTGAAAGCTCTGTAGCACCAGTACCAACCCCATCAA
GCTCTACTACTGAAAGCTCTTCTGCTCCAGTACCAACTCCATCCAGCTCTA
CTACCGAAAGCTCTTCTACTCCAGTAACCAGCTCCACCACTGAAAGCTCT
GTAGCACCAGTACCAACCCCATCAAGCTCTACTACTGAAAGCTCTTCTGC
TCCAGTATCCAGCTCTACTACTGAAAGCTCTGTAGCACCAGTACCAACCC
CATCTTCCTCTAGCAACATCACTTCCTCTGCTCCATCTTCAACTCCATTCA
GCTCTAGCACTGAAAGCTCTGTAGCACCAGTACCAACCCCATCCAGCTCT
ACTACTGAAAGCTCTTCTGCTCCAGTATCCAGCTCCACCACTGAAAGCTC
TGTAGCACCAGTACCAACCCCATCTTCCTCTAGCAACATCACTTCCTCCGC
TCCATCTTCAACTCCATTCAGCTCTACTACTGAAAGCTTTTCTACTGGCAC
TACTGTCACTCCATCATCATCCAAATACCCTGGCAGTCAAACAGAAACCT
CTGTTTCTTCTACAACCGAAACTACCATTGTTCCAACTACAACTACGACTT
CTGTCACTACATCATCAACAACCACTATTACTACTACAGTTTGCTCTACAG
GAACAAACTCTGCCGGTGAAACTACTTCTGGATGCTCTCCAAAGACCATT
ACAACTACTGTTCCATGTTCAACCAGTCCAAGCGAAACCGCCTCGGAATC
AACAACCACTTCACCTACCACACCTGTAACTACAGTTGTCTCAACCACCG
TCGTTACTGCTGAGTATTCTACTAGTACAAAACCAGGTGGTGAAATTACA
ACTACATTTGTCACCAAAAACATTCCAACCACTTACCTAACCGCAATTGC
TCCAACTCCATCAGTCACTACGGTTACCAATTTCACCCCAACCACTATTAC
TACTACGGTTTGCTCTACAGGTACAAACTCTGCCGGTGAAACTACCTCTG
GATGCTCTCCAAAGACTGTCACAACCACTGTTCCTTGTTCAACTGGTACT
GGCGAATACACTACTGAAGCTACCACCCCTGTTACAACAGCTGTCACAAC
CACCGTTGTTACCACTGAATCCTCTACGGGTACTAACTCCGCTGGTAAGA
CGACAACTGGTCACACAACAAAGTCTGTACCAACCACCTATGTAACCACT
TTGGCTCCAAGTGCACCAGTAACTCCTGCCACTAATGCCATACCAACTAC

AATAACCACTACTGAATGTTCTGCTGCTACAAACGCTGCCGGTGAAACTA
CATCTGTATGCTCTGCTAAGACTATCGTAAGTTCTGCAAGCGCAGGCGAA
AACACCACCCCTGTCACGATAGCTATTCCAACCACAGTTGTTACCACTGA
GTCATCTGTTGGTACTAACTCCGCTGGCGAAACAACAACTGGTTACACAA
CCAAGTCCATCCCAACCACTTACATAACCACTTTGATTCCAGGTTCAAAT
GGTGCCAAGAATTACGAAACTGTGGCCACAGCAACCAACCCTATTTCAAT
CAAGACTACATCCCAACTAGCTACAACAGCTTCTGCTTCTAGCATGGCTC
CCGTTGTCACATCTCCATCTCTAACTGGTCCACTACAATCTGCTTCTGGTT
CTGCAGTCGCTACATACTCTGTTCCTTCTATCTCGAGTACTTACCAAGGTG
CTGCTAATATCAAGGTTCTTGGAAACTTTATGTGGTTGCTACTCGCTCTTC
CAGTTGTATTCTAA

## Claims

**1.** A process for conferring a capability to form a biofilm at the liquid-air interphase in fungi and yeasts, **characterized in that** it comprises the following steps:
a) removing proteins competing for the same actuation sites as Flo11p in the fungus or yeast;
b) increasing the amount of Flo11p protein or equivalents that the cell has by means of expression with a very powerful promoter, or by means of deletion of the elements limiting or inhibiting expression of its own promoter;
c) producing variants of the Flo11p protein or equivalents in the fungus or yeast.

**2.** A process for improving the capability to form a biofilm at the liquid-air interphase in fungi and yeasts, **characterized in that** it comprises the following steps:
a) removing proteins competing for the same actuation sites as Flo11p in the fungus or yeast;
b) increasing the amount of Flo11p protein or equivalents that the cell has by means of expression with a very powerful promoter, or by means of deletion of the elements limiting or inhibiting expression of its own promoter;
c) producing variants of the Flo11p protein or equivalents in the fungus or yeast.

**3.** A process according to claim 1, **characterized in that** step a) is omitted, and the two remaining steps b) and c) are carried out.

**4.** A process according to claim 2, **characterized in that** it begins in any of steps a), b) or c), and the remaining two steps not selected are omitted.

**5.** A process according to claims 1 and 2, **characterized in that** step a) is carried out by means of disruption of the FLO1 gene in a strain called MY138.

**6.** A process according to claims 1, 2 and 5, **characterized in that** step a) comprises the following steps:
- cloning the FL01-13 fragment into the EcoRV (KpnI-SacI) site of the pBSSK vector, generating plasmid pBSFLO1-13;
- cloning the FL01-42 fragment into the EcoRI/BamHI sites of the pBSSK vector, generating plasmid pBSFL01-42;
- extracting the FL01-42 fragment from plasmid pBSFL01-42 with the EcoRI and XbaI enzymes, and it was cloned into plasmid pBSFLO1-13 at the EcoRI and Xbal sites, generating plasmid pBSFLO1-1342;
- cloning the fragment corresponding to the Kan gene, flanked by the loxP sequences originating from plasmid pUG6, into the EcoRV site between the FL01-13 and FL01-42 regions of plasmid pBSFLO1-1342, generating the plasmid called pFLO1-1342-KanloxP;
- digesting the plasmid with BamHI, generating a fragment which is transformed into the MY138 strain, from which strain geneticin-resistant transformants are selected, and a PCR is carried out to check that the transformed fragment has been integrated and, therefore, that the FLO1 gene has been disrupted.
- removing the DNA fragments not pertaining to the yeast and obtaining a GRAS strain with double disruption of the FLO1 gene, called OSB101.

**7.** A process according to claims 1 and 2, **characterized in that** steps b) and c) are carried out by means of the construction of plasmid pFLT-FLO11 and producing a variant of this FLO11 protein present in the MY138 flor strain.

**8.** A process according to claims 1, 2 and 7, **characterized in that** the construction of plasmid pFLT-FLO11 confers the capability to form biofilms to strains lacking said capability.

**9.** A new yeast strain, **characterized by** its identification number, Saccharomyces cerevisiae / OSB101, and its order number CECT 11781.

**10.** A new yeast strain, **characterized by** its identification number, Saccharomyces cerevisiae / OSB102, and its order number CECT 11782.

**13.** The use of new strains of yeasts and fungi in the production of biologically aged sherry wine.

**14.** The use of new strains of yeasts and fungi in processes for separating yeasts from the culture medium in industrial processes.

**15.** The use of new strains of yeasts and fungi in water depuration processes.
